# EUROPEAN PATENT APPLICATION

(11) **EP 3 181 686 A1**
(43) Date of publication of application: **21.06.2017**
(21) Application number: 15829691.3
(22) Date of filing: 04.08.2015
(51) Int. Cl.: C12N 5/0775, C12N 5/095

(54) **CULTURE MEDIUM AND METHOD FOR ENRICHING AND MAINTAINING CANCER STEM CELLS (CSCS) USING SAID MEDIUM**

(30) Priority: 04.08.2014 ES 201400666
(71) Applicant: Universidad de Granada, E-18071 Granada (ES); Universidad de Jaén, 23071 Jaén (ES); Servicio Andaluz de Salud, 41071 Sevilla (ES)
(72) Inventor: MARCHAL CORRALES, Juan Antonio, E-18071 Granada (ES); JIMÉNEZ GONZÁLEZ, Gema, E-18071 Granada (ES); MORATA TARIFA, Cynthia, E-18071 Granada (ES); GARCÍA CHAVES, María Ángel, E-41071 Sevilla (ES); PERÁN QUESADA, Macarena, E-23071 Jaén (ES)
(74) Representative: Hoffmann Eitle
(86) International application number: PCT/ES2015/070606
(87) International publication number: WO 2016/020572

(57) **Abstract**

The present invention relates to a serum-free conditioned medium that solves the drawbacks mentioned in the prior art, as it does not require prior handling of the cells, and it furthermore allows starting from a large population with no additional cost. This medium favors *in vitro* proliferation and conservation of the pluripotency potential that allows maintaining a state that is undifferentiated with respect to the subpopulation of cancer stem cells (CSCs) and in turn does not allow survival of the differentiated cells.

## Description

### TECHNICAL FIELD

The present invention is comprised in the field of healthcare and relates to a new serum-free conditioned medium favoring *in vitro* proliferation and conservation of the pluripotency potential that allows maintaining a state that is undifferentiated with respect to the subpopulation of cancer stem cells (CSCs), and in turn does not allow survival of the differentiated cells.

### PRIOR ART

There are two methodologies used today for enriching a population with cancer stem cells (CSCs).

First, CSCs can be enriched using magnetic columns (MACS, magnetic-activated cell sorting). This methodology requires a surface marker that is characteristic of the desired population of CSCs, which will be labeled with a specific antibody, and after this the cells are passed through a strong magnetic field that may make a positive or negative selection of the labeled cells, obtaining separate populations [Dou J, Pan M, Wen P, Li, and Tang Q, Chu L, Zhao F, Jiang C, Hu W, Hu K, Gu N. Isolation and identification of cancer stem-like cells from murine melanoma cell lines. Cell Mol Immunol. 2007; 4(6):467-72.].

The second methodology is based on the use of a cytometer discriminating between fluorescence-labeled cells (FACS: fluorescence-activated cell sorting). Unlike in the magnetic column methodology, both surface markers and intracellular markers that allow sorting the labeled cells from the original pool can be used for FACS, likewise obtaining one positive population and another negative population [Croker AK, Goodale D, Chu J, Postenka C, Hedley BD, Hess DA, Allan AL. High aldehyde dehydrogenase and expression of cancer stem cell markers selects for breast cancer cells with enhanced malignant and metastatic ability. J Cell Mol Med. 2009; 13(8B):2236-52].

Enriching the subpopulation of CSCs using MACS or FACS requires prior labeling and the cells have to go through a series of different equipment to be able to obtain the final population, which means that the cells are damaged and that a large proportion of them die during the process. On one hand, these methodologies require an enormous economic investment for the machinery used, and in many cases it is not available, and on the other hand, due to the problem of cell survival it is necessary to start from a very large original "pool" in order to obtain a very small population in a non-optimal state.

Therefore, there is a need to identify a new medium that allows enriching a subpopulation of CSCs and solves the problems mentioned above.

### BRIEF DESCRIPTION OF THE INVENTION

The present invention provides new serum-free conditioned media favoring *in vitro* proliferation and conservation of the pluripotency potential of CSCs and maintaining these cells in an undifferentiated state, which in turn does not allow survival of the differentiated cells.

### BRIEF DESCRIPTION OF THE DRAWINGS

**Figure 1****.** A) Aldehyde dehydrogenase activity. B) Multidrug resistance transporters (Hoescht). C) Sphere formation (15X).
**Figure 2.** Diagram representing the established culture conditions.
**Figure 3****.** A) Phenotypic characterization of HCT 116. B) Phenotypic characterization of A375. C) Spheres formed in HCT116 (20X lens). D) Spheres formed in A375 (20X lens).
**Figure 4****.** Phenotypic study for CSC markers in fresh colon tumors.
**Figure 5.** A) Cell cycle of HCT 116. B) Cell cycle of A375. C) Apoptosis of HCT 116. D) Apoptosis of A375.
**Figure 6****.** Proliferation assay of the cultures in suspension for HCT 116 (A) and A375 (B).
**Figure 7****.** Study of pluripotency gene expression in line HCT 116.

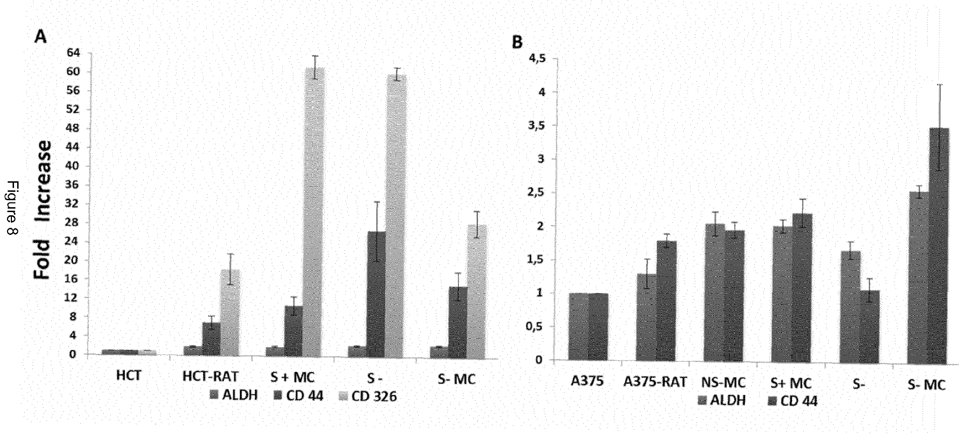

**Figure 8****.** Phenotypic markers for CSCs of the tumors obtained in mice with different culture conditions.
**Figure 9.** Increase in CSC markers of line HCT 116 in culture with different percentages of concentrated conditioned medium.
**Figure 10****.** Aldehyde dehydrogenase activity for the culture of HCT 116 with the different cytokines obtained from the Luminex. Final concentration of cytokine in the culture medium: 10 ng/ml.
**Figure 11****.** Aldehyde dehydrogenase activity for the culture of HCT 116 with the different combinations of cytokines. Final concentration of cytokine in the culture medium: 10 ng/ml.

### DETAILED DESCRIPTION OF THE INVENTION

### Definitions

Throughout the present invention, *"conventional medium"* will be understood as a standard cell culture medium. By way of example, said medium includes, but is not limited to, Eagle's Basal Medium (EBM), minimum essential medium (MEM), Dulbecco's Modified Eagle's Medium (DMEM), medium 199, Ham F-10, Ham F-12, McCoy's 5A, Dulbecco's MEM/FI2, RPMI 1640 medium, and Iscove's Modified Dulbecco's Medium (IMDM), 10% FBS (fetal bovine serum and 1% penicillin/streptomycin).

Each of the components of the conventional medium is defined below:
1. Eagle's Basal Medium (EBM): Basal medium with only essential amino acids. EBM contains eight vitamins B, the ten essential amino acids, plus cystine, tyrosine and glutamine. It was developed for culturing HeLa cells and fibroblasts. EBM modifications have resulting in other media, such as MEM and DMEM.
2. Minimum essential medium (MEM): This is the most widely used medium. It is obtained as a result of adding more amino acids and at a higher concentration than in EBM.
3. Dulbecco's Modified Eagle's Medium (DMEM): Contains four times the concentration of amino acids and vitamins than EBM does.
4. Medium 199: This is a mixture of salts enriched with amino acids, vitamins and other essential components for cell growth. It is widely used for culturing undifferentiated cells and for studying chromosome disorders as it is poor in folic acid.
5. Ham F-10: This medium contains metals such as Fe, Cu, Zn. Compared with other basal media, F-10 contains a wider range of components, including zinc, hypoxanthine, and thymidine. It is useful for culturing amniotic cells.
6. Ham F-12: This medium contains a wide range of components, including zinc, putrescine, hypoxanthine, and thymidine. It is normally used in serum-free form for c culturing Chinese hamster ovary (CHO) cells and supplemented with serum for any other cell type.
7. McCoy's 5A: McCoy medium was initially developed for human lymphocyte propagation, then successive modifications were performed until obtaining the current formula referred to as McCoy's 5a and it is used for growing both rat and human diploid cell lines.
8. Dulbecco's MEM/FI2: This is a 1:1 mixture of DMEM and Ham F-12. This formulation combines high DMEM concentrations of glucose, amino acids and vitamins, with the wide range of components of F-12. DMEM/F-12 does not contain proteins, lipids, or growth factors.
9. RPMI 1640 medium: This medium is unique and distinguished from the other media because it contains the reducing agent, glutathione, and high concentrations of vitamins. It also contains biotin. This medium is designed for growing lymphoblasts and leukemia cell lines in suspension. It has a wide range of applications with suitable supplements.
10. Iscove's Modified Dulbecco's Medium (IMDM): This is a very complete medium including in its formulation bovine albumin, transferrin, selenite, etc. It is very useful for culturing lymphocytes in serum-free medium. It also serves for other cell types, but in that case it requires serum at low concentrations.
11. FBS (fetal bovine serum): The serum provides hormones and growth factors. The bovine serum is the most widely used, but there are other types, such as calf serum (CF), horse serum (HS) and human serum (HuS).
12. Penicillin/streptomycin: Antimicrobial combination for inhibiting the growth of contaminants. The addition of antibiotics must be strictly controlled to prevent harmful effects on the culture.

Throughout the present invention, "*CSCs*" will be understood as a subpopulation of cells with characteristics similar to the stem cells, referred to as "tumor stem cells", "tumor-initiating cells" or "cancer stem cells." The characteristics they share in common with stem cells are: indefinite self-replication, asymmetric cell division, and resistance to toxic agents, in part due to the high expression of the ABC transporters. Furthermore, they are also characterized by having genetic instability (chromosomal and microsatellites), changes in chromatin, transcription and epigenetics, as well as in cell resource mobilization and in interactions with the microenvironment.

Throughout the present invention, "substantially pure population" will be understood as a population of cells where the cancer stem cells (CSCs) constitute at least 80% of the total cells in the population, preferably at least 85, 90, 95, 96, 97, 98 or 99% of the total cells in the population.

Throughout the present invention, "mesenchymal stem cells" (MSCs) will be understood as a multipotent stromal cell, originating from the mesodermal germ layer, which can differentiate into a range of cell types, including osteocytes (bone cells), chondrocytes (cartilage cells) and adipocytes (fat cells). The markers expressed by mesenchymal stem cells include CD105 (SH2), CD73 (SH3/4), CD44, CD90 (Thy-1), CD71 and Stro-1, as well as adhesion molecules CD106, CD166, and CD29. Included among negative markers for MSCs (unexpressed) are hematopoietic markers CD45, CD34, CD14, and costimulatory molecules CD80, CD86 and CD40, as well as adhesion molecule CD31. The MSCs can be obtained from, without being limited to, bone marrow, adipose tissue (such as subcutaneous adipose tissue), liver, spleen, testicles, menstrual blood, amniotic fluid, pancreas, periosteum, synovial membrane, skeletal muscle, dermis, pericytes, trabecular bone, human umbilical cord, lung, dental pulp and peripheral blood. The MSCs according to the invention can be obtained from any of the aforementioned tissues, such as from bone marrow, subcutaneous adipose tissue or umbilical cord. MSCs can be isolated from bone marrow by means of methods known by one skilled in the art. In general, said methods consist of isolating mononuclear cells by means of density gradient centrifugation (Ficoll, Percoll) of bone marrow aspirates, and subsequently seeding the isolated cells in tissue culture plates in medium containing fetal bovine serum. These methods are based on the capacity of MSCs to adhere to plastic, such that while the non-adherent cells are withdrawn from the culture, adhered MSCs can be expanded in culture plates. The MSCs can also be isolated from subcutaneous adipose tissue following a similar method, known by one skilled in the art. A method for isolating MSCs from bone marrow or from subcutaneous adipose tissue has previously been described (De la Source et al., Exp. Cell Res. 2004, Vol. 297: 313:328). In a particular embodiment of the invention, the mesenchymal stem cells are obtained from umbilical cord, preferably from human umbilical cord.

Throughout the present invention, "*spheres medium"* will be understood as an FBS-free medium useful for a culture in suspension, using DMEM-F12 medium (Dulbecco's Modified Eagle's Medium/Nutrient Mixture F-12 Ham Sigma-D8437), FGF and EGF, and wherein the remainder of components can vary depending on the cell type being cultured.

Throughout the description reference is made to the term "1X B27" as a supplement, such as B-27® Supplement, for example, at 1X concentration, for the serum-free cultures.

Throughout the present invention, *"confluence"* will be understood as the percentage of the culture surface taken up by cells.

The term "*culture*" or "*cell culture"* used in the present invention refers to the growth of cells or tissues in a suitable medium. In the present invention, said cell culture refers to an *in vitro* growth of the cells. In such cell culture, the cells proliferate but are not organized in the tissues *per se.*

Standard incubation conditions for cell proliferation are known by one skilled in the art. Such conditions comprise a temperature of 37°C, moisture saturation and atmosphere containing from 5% to 21% of O₂, 5% CO₂.

Other culture conditions suitable for expanding stem cells for use in the method of the present invention can be determined using standard methods known by one skilled in the art.

The term "*proliferation*" or "*expansion*" in the context of the present invention is used to refer to an increase in cell number derived from cell division.

In the context of the present invention, are used the following acronyms which refer to the following compounds:
1. IFNβ (Interferon type β)
2. IFNγ (Interferon type γ)
3. IL10 (Interleukin 10)
4. IL2 (Interleukin 2)
5. IL4 (Interleukin 4)
6. IL5 (Interleukin 5)
7. IL6 (Interleukin 6)
8. IL8 (Interleukin 8)
9. IL12 (Interleukin 12)
10. IL23 (Interleukin 23)
11. EGF (Epidermal Growth Factor)
12. FGF (Fibroblastic Growth Factor)
13. HGF (Hepatocyte Growth Factor)
14. GMCSF (Granulocyte and Monocyte Colony-stimulating Factor)
15. MCSF (Macrophage Colony-stimulating Factor)
16. PDGF-BB (Platelet-derived Growth Factor composed of two B chains (-BB)).
17. TNFα (Tumor Necrosis Factor α)
18. PIGF1 (Placental Growth Factor)
19. VEGF (Vascular Endothelial Growth Factor)

Unless expressly specified otherwise, the term "*comprising*" is used in the context of the present document to indicate that additional elements can optionally be present in addition to the elements of the list introduced by "*comprising*". However, it is contemplated as a specific embodiment of the present invention that the term "*comprising*" encompasses the possibility that additional elements are not present, i.e., for the purpose of this embodiment "*comprising*" must be understood as having the meaning "*consisting of*".

The term *"about*" refers to variations of +/- 1 % of the indicated value.

### DESCRIPTION

The present invention provides a serum-free conditioned medium solving the problems mentioned in the background of the invention, since it does not require prior handling of the cells, and furthermore, it can start a small population without any additional cost. This medium favors *in vitro* proliferation and conservation of the pluripotency potential that allows maintaining a state that is undifferentiated with respect to the CSC subpopulation and in turn does not allow survival of the differentiated cells.

In this sense, to obtain said medium the authors of the present invention have used mesenchymal stem cells (MSCs) from lipoaspirates. The cells were seeded in 75 cm² culture flasks at 40% confluence in conventional medium (DMEM, 10% FBS, 1% penicillin/streptomycin); after 24 hours they were washed with PBS (phosphate buffered saline) to remove any remainder of medium or serum, and was added sphere medium (DMEM-F12; 1% streptomycin-penicillin; 1 µg/mL hydrocortisone; 4 ng/mL heparin; 10 µg/mL insulin; 1X B27; 10 ng/mL EGF; 20 ng/mL FGF). The medium was collected every 48 hours and fresh sphere medium was added to it until it reached 80-90% confluence and the cells were discarded. The obtained medium was called serum-free conditioned medium (MC) and was passed through a 0.22 µM filter and kept at -20°C until use.

Once the serum-free conditioned medium was obtained, it was frozen as explained in the preceding paragraph; then the authors obtained both established tumor cell lines and tumor cell lines coming from primary cultures of samples from cancer patients, said cells being cultured in a suitable medium. A first differential trypsinization was performed on these cells. In this sense, after withdrawing the medium, the primary cultures of samples from cancer patients were washed twice with PBS. 2.5 mL of diluted trypsin (T4049 sigma) at 0.05% in PBS were added, said cells being incubated with the trypsin for a period of 2 minutes at 37°C, the enzyme subsequently being inactivated with FBS, adding the FBS slowly and preventing it from falling directly onto the cells to prevent them from becoming detached due to mechanical action. Trypsin-sensitive cells (TS) were thereby obtained. These obtained trypsin-sensitive cells (TS) were centrifuged and seeded in the serum-free conditioned medium (MC) of the invention.

Subsequently, the cells that were adhered in the plate were washed with PBS, a second trypsinization being carried out with the diluted trypsin described above. The cells were incubated for 3 minutes (time varying depending on the cell line) at 37°C, the trypsin was inactivated with FBS and these cells were discarded. The plate was washed again with PBS twice to completely remove the cells in suspension and the remainders of serum. Finally, the remaining cells, referred to as trypsin-resistant cells (TR), became completely detached with 0.25% trypsin.

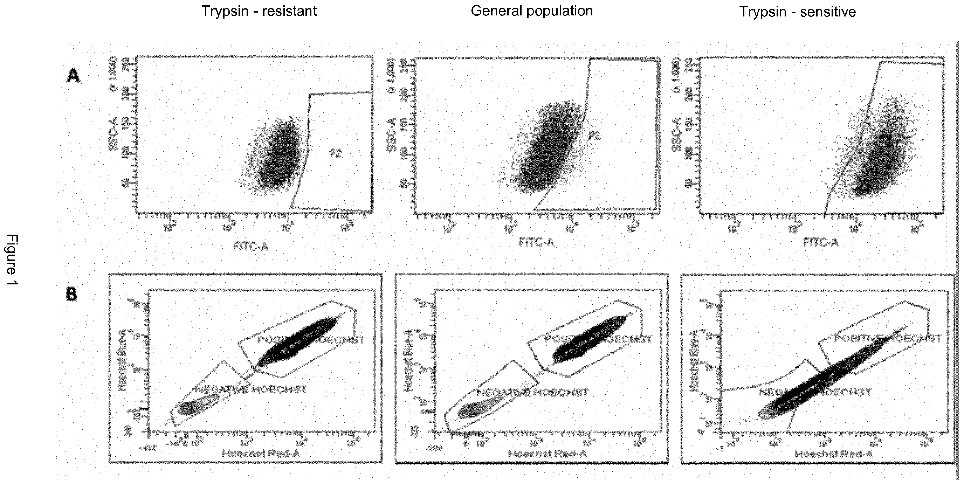

It was then verified by means of phenotypic and functional characterization that the cells obtained in the first trypsinization had a higher percentage of CSCs, and that they are resistant to a subpopulation that is more differentiated than the general population. For said phenotypic characterization, aldehyde dehydrogenase activity was studied (ALDEFLUOR® Kit, Stem Cell 01700), demonstrating that it is higher in the subpopulation of TS cells (94.35±1.77%) and lower in the TR cells (0.4±%) compared with the general population (7.55±0.78%) (Figure 1A). With respect to the "*side population*", which represents the population with the capacity to expel Hoescht 33342 (B2261 Sigma), and therefore expresses multidrug resistance (MDR) transporters, said population greatly increases in TS cells (70.50±4.38%) compared to TR cells (8.05±0.35) and to the general population (8.75±0.64) (Figure 1 B).

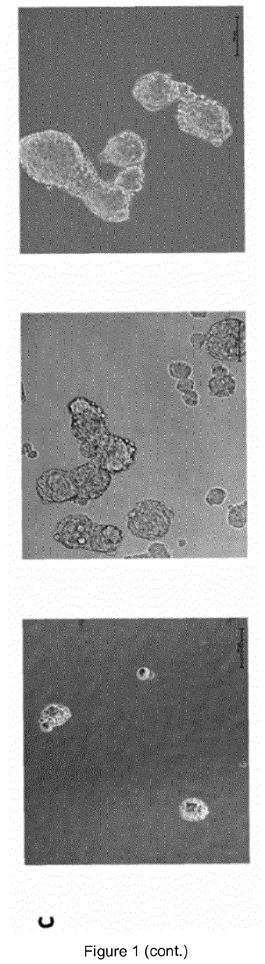

The capacity to form spheres after 6 days in low-adherence plates and conventional sphere medium (DMEM:F12, penicillin/streptomycin, B27, ITS, hydrocortisone, heparin, EGF, FGF) is likewise greater in TS cells (213.50±2.12) compared to TR cells (71.5±0.71) and the general population (134.00 ±38.18), furthermore being of a smaller size in the latter two (Figure 1 C).

These results clearly show a higher proportion of CSCs in the trypsin-sensitive population (TS).

Therefore, a first aspect of the invention relates to a method for enriching in cancer stem cells (CSCs) as well as obtaining a substantially pure population of cancer stem cells (CSCs), comprising the following steps:
a. Obtaining a biological sample comprising a cell culture of cancer stem cells (CSCs) adhered to a plate, vessel or flask suitable for culturing cells;
b. Withdrawing the culture medium from the biological sample from step a) and optionally washing the cell culture;
c. Adding trypsin, preferably diluted between 0.01 and 0.25%, more preferably diluted between 0.01 and 0.1%, more preferably diluted about 0.05%, to the primary culture from step b) and incubating said cells with the trypsin;
d. Inactivating the trypsin; and
e. Obtaining those cells that have become detached from the medium due to the action of trypsin.

In a preferred embodiment of the first aspect of the invention, after withdrawing the culture medium in step b), the cell culture is washed with PBS. In another preferred embodiment, the enzyme in step d) is inactivated by adding FBS, which is added slowly and prevented from falling directly onto the cells to prevent said cells from becoming detached due to mechanical action. It is noteworthy that the percentage of trypsin (to obtain the "differential trypsiniation") is relevant. The trypsin is added, diluted in PBS, preferably diluted to a percentage between 0.01 and 0.1%, more preferably diluted to about 0.05%

The population of cells with trypsin-sensitive cells (TS) obtained from the method of the first aspect of the invention is enriched with cancer stem cells (CSCs). Therefore, a second aspect of the invention relates to a population of cells comprising cancer stem cells (CSCs) obtained by the method of the first aspect of the invention. Likewise, a preferred embodiment of the second aspect of the invention relates to a substantially pure population of cells comprising cancer stem cells (CSCs) obtained by the method of the first aspect of the invention.

Once it is shown that cancer stem cells (CSCs) are more sensitive to the action of trypsin, the authors of the present invention obtained established colon tumor cell line (HCT 116) and melanoma tumor cell line (A375). Said lines were cultured in a conventional medium until reaching a confluence of 80-90%. After this point, the cells are sorted by means of flow cytometry (cell sorting) according to the aldehyde dehydrogenase activity (ALDEFLUOR® Kit, Stem Cell 01700) as a CSC marker. Two different populations were obtained with this technique, i.e., the cells positive for the mentioned enzyme (CSCs also referred to as "Positive sorter cells (S+)") and the negative cells (differentiated cells also referred to as "Negative sorter cells (S-)"). Then three types of culture conditions were established for each cell type:
Positive sorter cells (S+):
   - HCT-A375 S+: low-adherence plates and sphere medium.
   - HCT-A375 S+ T: low-adherence plates, sphere medium and a "transwell" chamber on which MSCs were seeded 48 hours prior.
   - HCT-A375 S+ MC: low-adherence plates and conditioned medium.
Negative sorter cells (S-):
   - HCT-A375 S-: low-adherence plates and sphere medium.
   - HCT-A375 S- T: low-adherence plates, sphere medium and a "transwell" chamber on which MSCs were seeded 48 hours prior.
   - HCT-A375 S- MC: low-adherence plates and conditioned medium.

The same day cell sorting was performed a general population culture flask was trypsinized, and after washing the cells twice with PBS to remove any remainder of conventional medium they were seeded in low-adherence plates and sphere medium (population referred to as NS). After this first phase, at the same time as the positive and negative populations, this population was split up to establish three different culture conditions:
- HCT-A375 NS: low-adherence plates and sphere medium.
- HCT-A375 NS T: low-adherence plates, sphere medium and a "transwell" chamber on which MSCs were seeded 48 hours prior.
- HCT-A375 NS MC: low-adherence plates and conditioned medium.

This latter population provides information about how the different culture conditions affect the proportion of CSCs without having to perform a prior cell sorting.

Finally, a control population was established where the cells were cultured in adherence:
- HCT-A375: culture in adherence and conventional medium.
- HCT-A375 T: culture in adherence, conventional medium and a "transwell" chamber on which MSCs were seeded 48 hours prior.
- HCT-A375 MC: culture in adherence and conditioned medium. 24 hours after seeding the cells in the wells, they are washed twice with PBS and conditioned medium is added.

Every 48-72 hours the formed spheres were broken up to prevent them from becoming necrotic. To that end, the cells were collected and centrifuged, and subsequently they were incubated for 5 minutes at 37°C with trypsin, inactivating said enzyme with serum and washing with PBS to remove any remainder of serum after inactivation. The cells were then placed in the three low-adherence and serum-free medium culture conditions again.

Aldehyde dehydrogenase activity was studied for the phenotypic characterization, and characteristic CSC markers of each tumor type, such as CD326 and CD44 for cell line HCT 116 and CD44 for cell line A375, were furthermore used. Two weeks later, it could be seen how in the control populations of both lines there was a significant increase in the three markers, this being more evident in cultures using the serum-free conditioned medium of the present invention, such as HCT MC and HCT NS MC, where the percentage of these cells doubled.

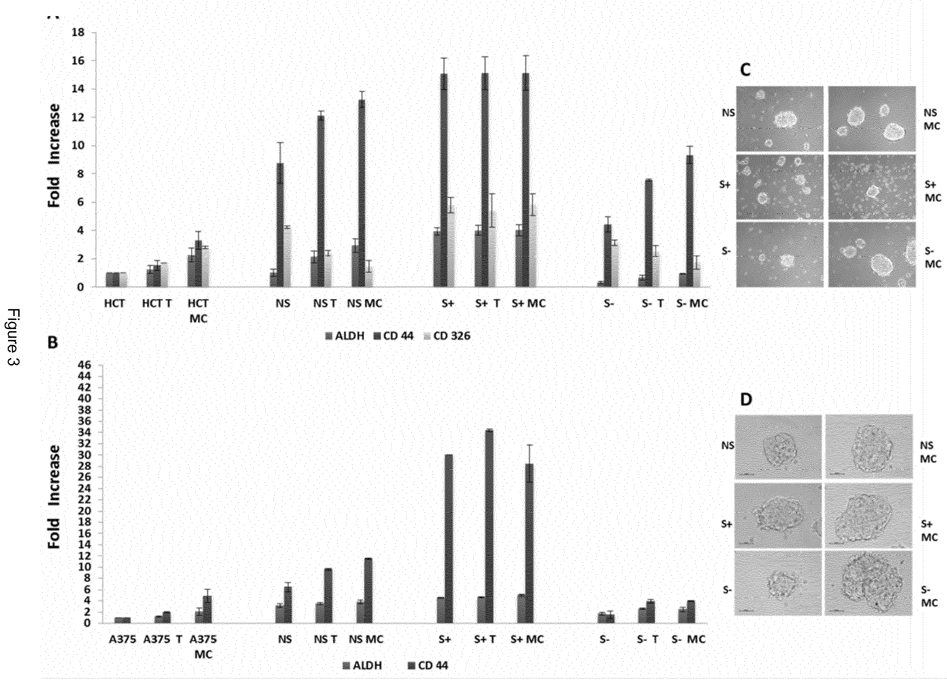

The positive sorter cells (S+) maintained their characteristics after two weeks, and the negative sorter cells (S-) gradually acquired characteristics of CSCs, the increase being more significant with the culture with conditioned medium (S-MC) (Figure 3 A-B), so this medium favors the phenotype of the progenitor cells that are starting to differentiate not reverting to CSCs.

These results are consistent with the size of the spheres formed in the different culture conditions, being larger in the culture with conditioned medium (Figure 3 C-D).

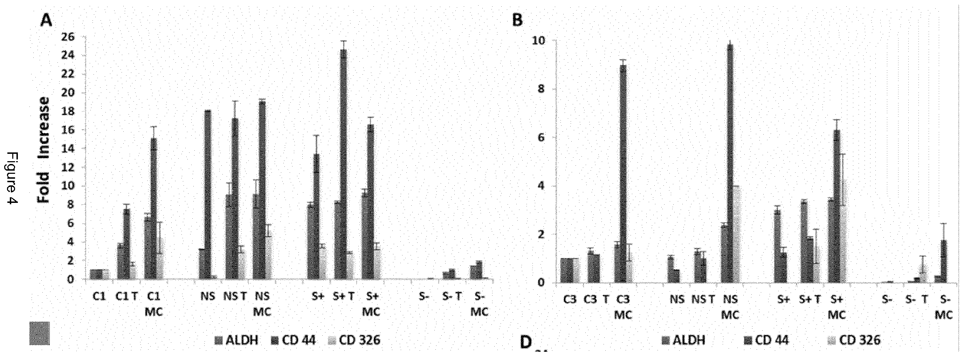

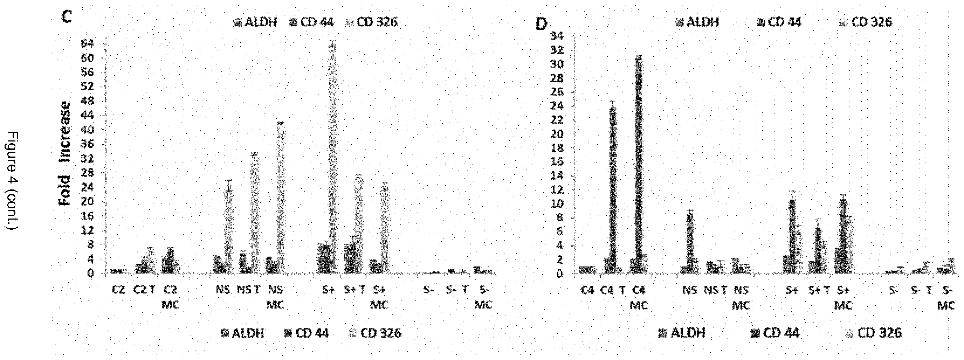

This same experiment was performed with fresh colon tumors from different patients and in lowpass (P4) and long-pass (P8), the obtained results being equivalent to those obtained in the established tumor cell lines. In fact, it was in the control populations with conditioned medium where there was greater CSC enrichment (C MC and NS MC), the positive sorter population (S+) maintained the enrichment and the negative sorter population (S-) in the culture in sphere medium showed very little or no labeling, said labeling increasing with use of the conditioned medium (Figure 4).

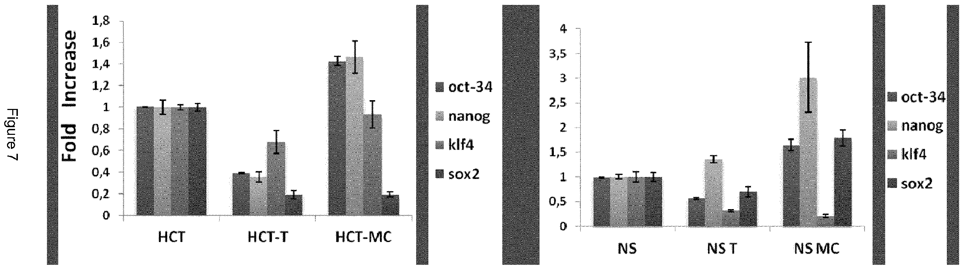

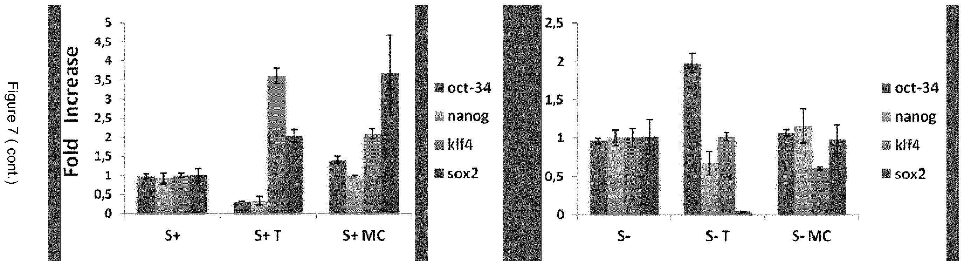

A genetic study was also conducted for cell line HCT 116, where the genes were analyzed for pluripotency (Nanog, Sox 2, Oct 3-4 and KLF4). It can be seen in the results how expression of at least three of the genes increases in the control cultures in which conditioned medium (HCT MC and HCT NS MC) was used. In the positive sorter cell population with conditioned medium (S+ MC), there is a significant increase in the expression of the four genes, and even in the negative sorter cell population (S- MC), where phenotypic markers for CSCs have been emerging, there is a significant increase for the Oct 3-4 gene (Figure 7).

Finally and to complete this phase of the study, an *in vivo* assay was conducted with immunosuppressed mice. First the cells were cultured for 12 days in the following conditions:
- HCT-A375 RAT: culture in adherence and conventional medium.
- HCT-A375 NS MC: low-adherence plates and conditioned medium.
- HCT-A375 S+ MC: low-adherence plates and conditioned medium.
- HCT-A375 S-: low-adherence plates and sphere medium.
- HCT-A375 S- MC: low-adherence plates and conditioned medium.

After this time, the cultures were trypsinized and a suspension of PBS was inoculated with 10,000 cells. Once the tumor developed, it was removed and broken down mechanically and enzymatically with collagenase I, collagenase IV and dispase for 30 minutes at 37°C. The cells obtained with this process were placed in culture in normal adherence culture flasks and with conventional culture medium (DMEM, 10% FBS, 1% penicillin/streptomycin). One week into the culture, cytometry was performed to check for characteristic CSC markers of each cell line studied up until now. It could be seen in the results how the markers gradually increased as the culture conditions became more restrictive for CSCs, and it is surprising how the negative sorter cells, which were in conditioned medium before being inoculated in the mouse, presented one of the highest enrichments. This result is consistent with the results of the phenotypic labeling experiments after 12 days in culture (Figure 3 and 4) and with the expression of pluripotency genes (Figure 7), indicating that the conditioned medium favors regression to CSCs of the progenitor cells that were in the first phases of differentiation, and it favors them proliferating to restore a tumor in the mouse.

Therefore, a third aspect of the invention relates to a method for obtaining a cell culture medium, particularly a culture medium useful for isolating and/or enriching cancer stem cells (CSCs) and/or for obtaining a substantially pure population of cancer stem cells (CSCs), comprising the following steps:
a. Seeding mesenchymal stem cells (MSCs) in a plate, vessel or flask suitable for culturing cells using a conventional culture medium (as defined in the paragraph of definitions), where the confluence of said cells is preferably between 40 and 90%, more preferably between 40 and 80%, even more preferably about 40% at the start of the process;
b. Optionally removing any remainder of medium and/or serum from the culture medium from step a), preferably the washing process is performed 24 hours after the start of the process;
c. Adding a sphere medium or conventional medium without FBS (as defined in the paragraph of definitions) to the product of step b);
d. Collecting the culture medium from step c), where the culture medium will preferably be collected at least 48 hours after adding the medium in step c), preferably between 48 and 72 hours and more preferably every 48 hours, and adding new sphere medium;
e. Repeating the process from step d) until the cells in culture reach a level of confluence of 80-90%;
f. Optionally filtering, preferably using a 0.22 µM filter, and freezing the medium obtained in steps d) and e) until use.

The mesenchymal stem cells (MSCs) used in step a) can come from any suitable source for obtaining mesenchymal stem cells. Preferably, the MSCs are from the stroma of breast cancer tumors, umbilical cord or lipoaspirates, the MSCs more preferably coming from lipoaspirates.

Preferably, at the start of the process of step a) the cell seeding confluence will be at about 40%, since below this margin there are very few cells and they will grow very slowly (MSCs need to be in contact with one another), and moreover, said cells will grow faster, reaching maximum confluence too quickly. This maximum confluence ranges from 80-90%, above which the cells release stress and apoptosis factors and cytokines that are harmful to the conditioned culture medium of the present invention.

In a preferred embodiment of step b) of the third aspect of the invention, remainders of serum or medium are removed by means of washing with PBS (phosphate buffered saline).

As described in detail in step d) of the method of the third aspect of the invention, the culture medium will preferably be collected at least 48 hours after adding the medium in step c), preferably between 48 and 72 hours and more preferably every 48 hours. This time period is relevant for the cells to release the number of factors and cytokines forming the serum-free conditioned medium (MC) of the present invention. Nevertheless, too much time can cause the medium to acidify, negatively affecting the cells.

Finally, the authors of the present invention have found that the obtained serum-free conditioned medium (MC) can be kept at -20°C until use for at least 6 months.

The cell-free medium that is finally obtained is referred to as serum-free conditioned medium (MC). Therefore, a fourth aspect of the invention relates to the serum-free conditioned medium (MC) obtained by the method described in the third aspect of the invention.

In a preferred embodiment of the fourth aspect of the invention, the conditioned medium is concentrated and obtainable using filters and/or centrifugation, preferably using special Millipore filters concentrating the medium 25-fold by means of centrifugation: Amicon Ultra-15 centrifugal filter units (3 kDa).

After the obtaining the serum-free conditioned medium (MC), the authors of the present invention analyzed said medium and identified a series of cytokines subsequently characterized as fundamental elements of the medium, thereby allowing artificial/synthetic production of a culture medium. Specifically, the serum-free conditioned medium contains, in addition to the typical components of the sphere medium, the following components: IFNβ, IFNγ, IL10, IL2, IL4, IL5, IL6, IL8, IL12, IL23, EGF, FGF, HGF, GMCSF, MCSF, PDGF-BB, TNFα, PIGF1 and VEGF. The result of the characterization of the serum-free conditioned medium of the present invention is shown below:

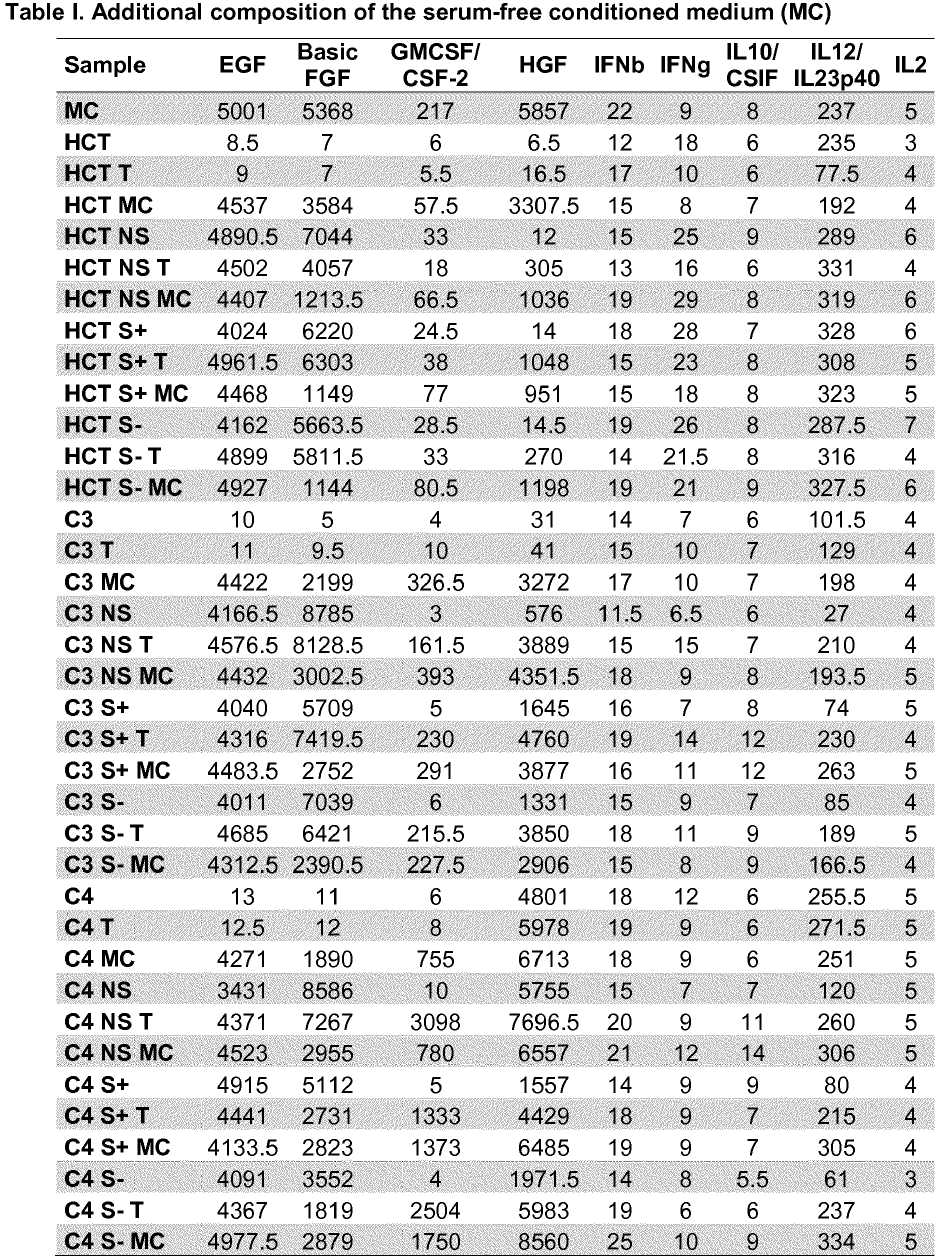

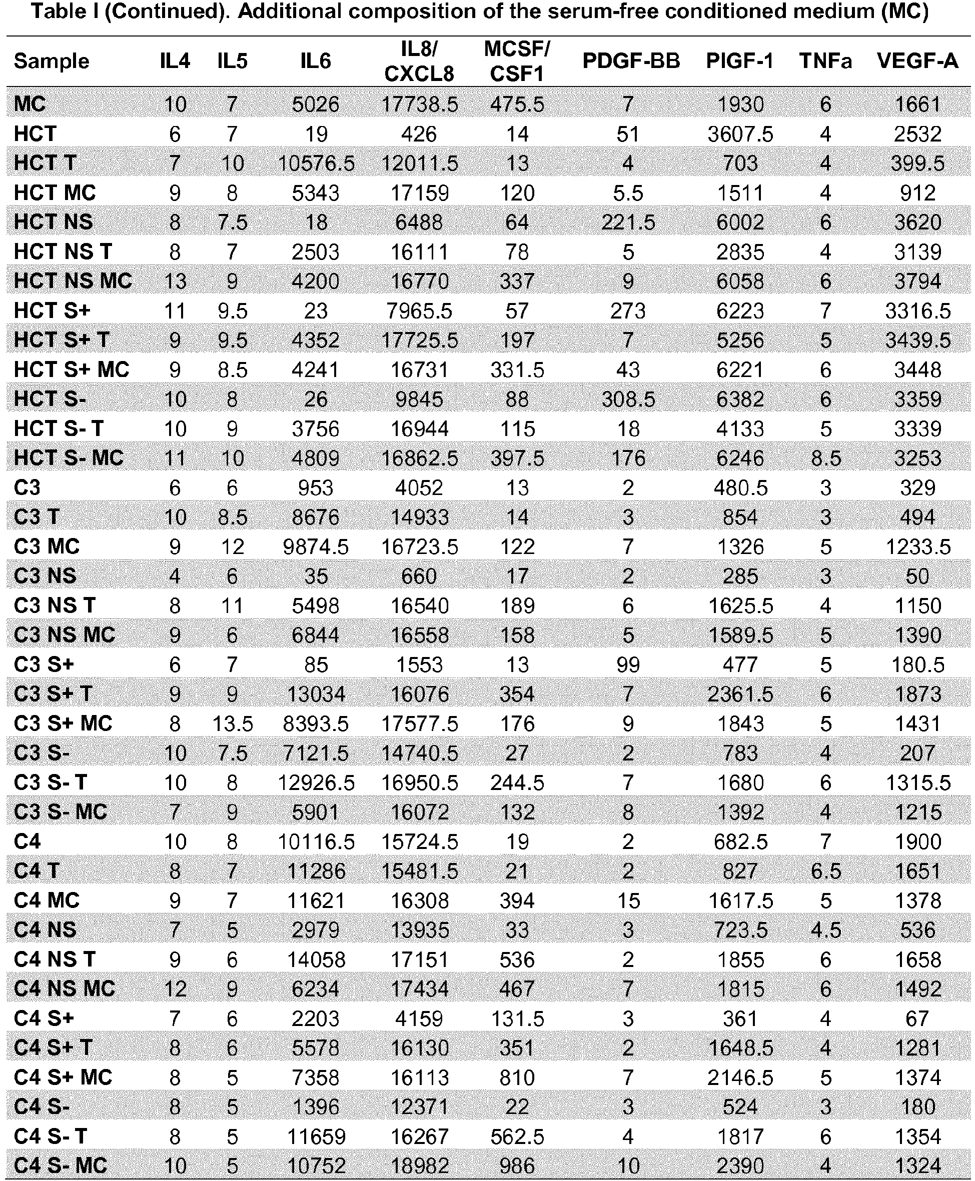

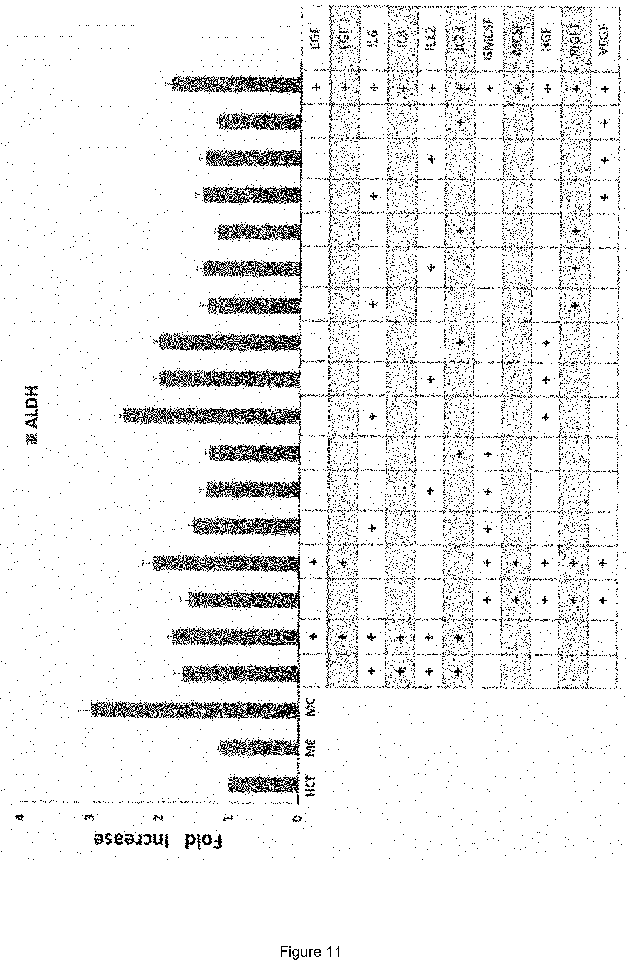

Therefore, based on use of the sphere medium EGF- and FGF-free, specifically in a medium containing the following elements: DMEM:F12, penicillin/streptomycin, B27, ITS, hydrocortisone and heparin, the authors of the present invention tested adding different combinations of the compounds present in the serum-free conditioned medium of the present invention, specifically 12 combinations were tested, as shown in Figure 11.

After 6 days in culture a test to determine aldehyde dehydrogenase activity was conducted and it could be seen that the worst two combinations (PIGF1-IL23 and VEGF-IL23) caused the exact same increase in the percentage of expression of ALDH1 with respect to the control population (HCT) as the usual combination of EGF and FGF (HCT ME). The remainder of combinations generated a much higher enrichment, but it is important to mention factor HGF, which when combined with IL6 caused a 2.5-fold increase with respect to the control population and the sphere medium and the combination of cytokines IL12 and IL23 that caused a two-fold increase with respect to the control population (HCT) and the sphere medium (HCT ME) (Figure 11).

Therefore, a fifth aspect of the invention relates to a natural and synthetic culture medium comprising a medium suitable for culturing CSCs, such as a conventional medium or a sphere medium, supplemented with at least one compound selected from the list consisting of: IFNβ, IFNγ, IL10, IL2, IL4, IL5, IL6, IL8, IL12, IL23, EGF, FGF, HGF, GMCSF, MCSF, PEGFBB, TNFα, PIGF1 and VEGF.

A preferred embodiment of the fifth aspect of the invention relates to a natural or synthetic culture medium comprising Eagle's Basal Medium (EBM), minimum essential medium (MEM), Dulbecco's Modified Eagle's Medium (DMEM), medium 199, Ham F-10, Ham F-12, McCoy's 5A, Dulbecco's MEM/FI2, RPMI 1640 medium, and Iscove's Modified Dulbecco's Medium (IMDM), 10% FBS (fetal bovine serum and 1% penicillin/streptomycin) and at least one compound selected from the list consisting of: IFNβ, IFNγ, IL10, IL2, IL4, IL5, IL6, IL8, IL12, IL23, EGF, FGF, HGF, GMCSF, MCSF, PEGFBB, TNFα, PIGF1 and VEGF.

Another preferred embodiment of the fifth aspect of the invention relates to a natural or synthetic culture medium comprising DMEM-F12 (Dulbecco's Modified Eagle's Medium/Nutrient Mixture F-12 Ham Sigma-D8437), and at least one compound selected from the list consisting of: IFNβ, IFNγ, IL10, IL2, IL4, IL5, IL6, IL8, IL12, IL23, EGF, FGF, HGF, GMCSF, MCSF, PEGFBB, TNFα, PIGF1 and VEGF.

Another preferred embodiment of the fifth aspect of the invention relates to a natural or synthetic culture medium comprising DMEM-F12; 1% streptomycin-penicillin; 1 µg/mL hydrocortisone; 4 ng/mL heparin; 10 µg/mL insulin; 1X B27, and at least one compound selected from the list consisting of: IFNβ, IFNγ, IL10, IL2, IL4, IL5, IL6, IL8, IL12, IL23, EGF, FGF, HGF, GMCSF, MCSF, PEGFBB, TNFα, PIGF1 and VEGF.

Another preferred embodiment of the fifth aspect of the invention relates to a natural and synthetic culture medium comprising a medium suitable for culturing CSCs, such as a conventional medium or a sphere medium, supplemented with at least one combination of compounds selected from the list consisting of:
1. IL6, IL8, IL12 and IL23;
2. IL6, IL8, IL12, IL23, EGF and FGF;
3. GMCSF, MCSF, HGF, PIGFI and VEGF;
4. EGF, FGF, GMCSF, MCSF, HGF, PIGFI and VEGF;
5. IL6 and GMCSF;
6. GMCSF and IL12;
7. GMCSF and IL23;
8. IL6 and HGF;
9. HGF and IL12;
10. HGF and IL23;
11. PIGFI and IL6;
12. PIGFI and IL12;
13. PIGFI and IL23;
14. VEGF and IL6;
15. VEGF and IL12;
16. VEGF and IL23;
17. IL6, IL8, IL12, IL23, EGF, FGF, GMCSF, MCSF, HGF, PIGFI and VEGF.

A preferred embodiment of the fifth aspect of the invention relates to a natural or synthetic culture medium comprising Eagle's Basal Medium (EBM), minimum essential medium (MEM), Dulbecco's Modified Eagle's Medium (DMEM), medium 199, Ham F-10, Ham F-12, McCoy's 5A, Dulbecco's MEM/FI2, RPMI 1640 medium, and Iscove's Modified Dulbecco's Medium (IMDM), 10% FBS (fetal bovine serum and 1% penicillin/streptomycin) and at least one combination of compounds selected from the list consisting of:
1. IL6, IL8, IL12 and IL23;
2. IL6, IL8, IL12, IL23, EGF and FGF;
3. GMCSF, MCSF, HGF, PIGFI and VEGF;
4. EGF, FGF, GMCSF, MCSF, HGF, PIGFI and VEGF;
5. IL6 and GMCSF;
6. GMCSF and IL12;
7. GMCSF and IL23;
8. IL6 and HGF;
9. HGF and IL12;
10. HGF and IL23;
11. PIGFI and IL6;
12. PIGFI and IL12;
13. PIGFI and IL23;
14. VEGF and IL6;
15. VEGF and IL12;
16. VEGF and IL23;
17. IL6, IL8, IL12, IL23, EGF, FGF, GMCSF, MCSF, HGF, PIGFI and VEGF.

Another preferred embodiment of the fifth aspect of the invention relates to a natural or synthetic culture medium comprising DMEM-F12 (Dulbecco's Modified Eagle's Medium/Nutrient Mixture F-12 Ham Sigma-D8437), and at least one combination of compounds selected from the list consisting of:
1. IL6, IL8, IL12 and IL23;
2. IL6, IL8, IL12, IL23, EGF and FGF;
3. GMCSF, MCSF, HGF, PIGFI and VEGF;
4. EGF, FGF, GMCSF, MCSF, HGF, PIGFI and VEGF;
5. IL6 and GMCSF;
6. GMCSF and IL12;
7. GMCSF and IL23;
8. IL6 and HGF;
9. HGF and IL12;
10. HGF and IL23;
11. PIGFI and IL6;
12. PIGFI and IL12;
13. PIGFI and IL23;
14. VEGF and IL6;
15. VEGF and IL12;
16. VEGF and IL23;
17. IL6, IL8, IL12, IL23, EGF, FGF, GMCSF, MCSF, HGF, PIGFI and VEGF.

Another preferred embodiment of the fifth aspect of the invention relates to a natural or synthetic culture medium comprising DMEM-F12; 1% streptomycin-penicillin; 1 µg/mL hydrocortisone; 4 ng/mL heparin; 10 µg/mL insulin; 1X B27, and at least one combination of compounds selected from the list consisting of:
1. IL6, IL8, IL12 and IL23;
2. IL6, IL8, IL12, IL23, EGF and FGF;
3. GMCSF, MCSF, HGF, PIGFI and VEGF;
4. EGF, FGF, GMCSF, MCSF, HGF, PIGFI and VEGF;
5. IL6 and GMCSF;
6. GMCSF and IL12;
7. GMCSF and IL23;
8. IL6 and HGF;
9. HGF and IL12;
10. HGF and IL23;
11. PIGFI and IL6;
12. PIGFI and IL12;
13. PIGFI and IL23;
14. VEGF and IL6;
15. VEGF and IL12;
16. VEGF and IL23;
17. IL6, IL8, IL12, IL23, EGF, FGF, GMCSF, MCSF, HGF, PIGFI and VEGF.

Another preferred embodiment of the fifth aspect of the invention relates to a natural and synthetic culture medium comprising a medium suitable for culturing CSCs, such as a conventional medium or a sphere medium, supplemented with the combination of compounds shown in Table I, preferably in the same proportion.

A preferred embodiment of the fifth aspect of the invention relates to a natural or synthetic culture medium comprising Eagle's Basal Medium (EBM), minimum essential medium (MEM), Dulbecco's Modified Eagle's Medium (DMEM), medium 199, Ham F-10, Ham F-12, McCoy's 5A, Dulbecco's MEM/FI2, RPMI 1640 medium, and Iscove's Modified Dulbecco's Medium (IMDM), 10% FBS (fetal bovine serum and 1% penicillin/streptomycin) supplemented with the combination of compounds shown in Table I, preferably in the same proportion.

Another preferred embodiment of the fifth aspect of the invention relates to a natural or synthetic culture medium comprising DMEM-F12 (Dulbecco's Modified Eagle's Medium/Nutrient Mixture F-12 Ham Sigma-D8437), supplemented with the combination of compounds shown in Table I, preferably in the same proportion.

Another preferred embodiment of the fifth aspect of the invention relates to a natural or synthetic culture medium comprising DMEM-F12; 1% streptomycin-penicillin; 1 µg/mL hydrocortisone; 4 ng/mL heparin; 10 µg/mL insulin; 1X B27, supplemented with the combination of compounds shown in Table I, preferably in the same proportion.

Another preferred embodiment of the fifth aspect of the invention relates to a natural or synthetic culture medium comprising any of the media described in the fifth aspect of the invention in concentrated form. Said concentrated medium is obtainable using filters and/or centrifugation, preferably using special Millipore filters concentrating the medium 25-fold by means of centrifugation: Amicon Ultra-15 centrifugal filter units (3 kDa).

A sixth aspect of the invention relates to a method for enriching CSCs in a cell culture comprising the following steps:
a) Seeding a biological sample comprising CSCs in the conditioned medium (MC) defined in either of the fourth or fifth aspects of the present invention; and
b) Obtaining the population of cells enriched in CSCs.

In a preferred embodiment of the sixth aspect of the invention, said method additionally comprises the method for enriching CSCs of the first aspect of the invention.

Particularly, the authors of the present invention propose performing, prior to the seeding of a biological sample comprising CSCs in the conditioned medium (MC) of the invention, the following steps:
a) A first trypsinization of the culture with diluted trypsin, preferably diluted between 0.01 and 0.25%, more preferably diluted between 0.01 and 0.1%, more preferably diluted to about 0.05%;
b) Optionally, a second trypsinization with diluted trypsin, preferably between 0.01 and 0.25%, more preferably diluted between 0.01 and 0.1%, more preferably diluted to about 0.05%; and
c) The seeding in the medium object of the invention according to the sixth aspect of the invention.

Each of the phases is described in further detail below:
First trypsinization of the culture with diluted trypsin. This process comprises the following steps:
   - Washing a medium-free population of cells (established cell lines or primary cultures) with PBS and adding diluted trypsin in PBS, preferably diluted between 0.01 and 0.25%, more preferably diluted (preferably in PBS) between 0.01 and 0.1%, more preferably diluted to about 0.05%;
   - Incubating the cells once with trypsin and at a suitable temperature, preferably about 2 minutes at 37°C;
   - Inactivating the trypsin preferably by means of FBS and centrifuging preferably at 1500 rpm for 5 min;
   - Collecting the "detached" cells referred to as trypsin-sensitive cells (TS1).
Second trypsinization with diluted trypsin. This process comprises the following steps:
   - The cells obtained in the first trypsinization are seeded in a culture flask, preferably for 24 h;
   - They are washed, preferably with PBS, and diluted trypsin is added, preferably diluted in PBS, more preferably diluted between 0.01 and 0.25%, more preferably diluted between 0.01 and 0.1%, more preferably diluted to about 0.05%,
   - Incubating the cells with the trypsin for a suitable time and at a suitable temperature, preferably about 2 minutes at 37°C;
   - Inactivating the trypsin preferably by means of FBS and centrifuging preferably at 1500 rpm for 5 min;
   - Collecting the "detached" cells, referred to as trypsin-sensitive cells (TS2).

Once these steps have ended, both trypsin-sensitive cells TS1 and TS2 are seeded in the conditioned medium (MC) of the invention (in some preferred embodiments of the present invention, only one of the two sensitive strains obtained can be seeded). It is noteworthy that each of the subpopulations of cells TS1 and TS2 has a higher percentage of CSCs.

Therefore, a seventh aspect of the invention relates to a method for enriching CSCs in a cell culture comprising the following steps:
a. A first trypsinization of the culture with diluted trypsin;
b. Optionally a second trypsinization with diluted trypsin; and
c. Seeding a biological sample comprising the cells obtained from step a) and/or b) in the conditioned medium (MC) defined in any of the fourth or fifth aspects of the present invention; and
d. Obtaining the population of cells enriched in CSCs.

In addition, an eighth aspect of the present invention relates to a cellular composition, a population of cells or a substantially pure population of cells obtained by the enrichment process method for enriching according to any of the first, sixth or seventh aspects of the present invention.

Therefore, based on the prior studies aimed at analyzing which culture condition is the most suitable for greater CSC enrichment, it can be concluded that the use of the conditioned medium is what offers the greatest advantages. In fact, the conditioned medium of the present invention allows obtaining a population with a higher enrichment percentage in a shorter time than the serum-free medium described in the prior art, reducing the time needed, and therefore the economic investment required, to obtain a population suitable for the conducting later assays.

In addition, the differential trypsinization methodology for enriching populations of CSCs based on the different adhesion capacity of the different subpopulations, CSCs at a low concentration of trypsin being more sensitive, allows combining the enriched population with the mentioned conditioned medium object of the invention, which thereby allows increasing the number of cells with CSC characteristics obtained in trypsinization.

As a result of a higher capacity of isolating and enriching subpopulations of CSCs without causing cell damage, which is more cost-effective, and the conservation of its phenotype for a long period of time, there are a number of industrial applications that can be developed as a result.
- The study of the differential genetic and phenotypic characteristics of these cells, which allows conserving these characteristics over time.
- The determination again specific biomarkers of these cells both in cell cultures and primary cultures of cancer patients, which would allow obtaining information about the progression of the disease and about the response to treatment.
- Since these cells are cause metastasis, relapses and resistance to drugs used today in clinical practice, based on in this prior study it is possible to develop drugs against them, which allows the search for specific targets.
- The possibility of carrying out analyses of the efficacy again therapeutic approaches that are selective for these cells using automated high-throughput screening (HTS) techniques, and based on new antitumor synthetic or natural drugs, immunotherapy, the use of gene therapy or ionizing radiation, providing the possibility of a more custom-tailored therapy.
- Today there are only three active clinical trials (clinicaltrials.gov) with the objective of identifying and characterizing subpopulations of CSCs with invasive capacity from the primary tumor, metastases and blood samples in different types of cancer (NCT01577511; NCT00923052; NCT01641003); therefore, this methodology could help to implement new clinical trials.

Therefore, a ninth aspect of the invention relates to the use of a cellular composition, a population of cells or a substantially pure population of cells obtained by the method for enriching according to any of the first, sixth or seventh aspects of the present invention for determining new specific biomarkers of cancer stem cells that allow obtaining information about the progression of the disease and of the response al treatment.

A tenth aspect of the invention relates to the use of a cellular composition, a population of cells or a substantially pure population of cells obtained by the method for enriching according to any of the first, sixth or seventh aspects of the present invention to determine the efficacy again therapeutic approaches selective for these cancer cells using automated high-throughput screening (HTS) techniques, and based on new synthetic or natural antitumor drugs, immunotherapy, use of gene therapy or ionizing radiation, providing the possibility of a more custom-tailored therapy.

The following examples provided below serve to illustrate the present invention without limiting it in any way.

### EXAMPLES

### Example 1. Obtaining the CSC enrichment medium (conditioned medium)

The cells were seeded in 75 cm² culture flasks at 40% confluence in conventional medium (DMEM, 10% FBS, 1% penicillin/streptomycin). After 24 hours they were washed with PBS to remove any remainder of medium or serum, and sphere medium (DMEM-F12; 1% streptomycin-penicillin; 1 µg/mL hydrocortisone; 4 ng/mL heparin; 10 µg/mL insulin; 1X B27; 10 ng/mL EGF; 20 ng/mL FGF) was added. The medium was collected every 48 hours and fresh sphere medium was added until reaching 80-90% confluence and the cells were discarded. The medium was passed through a 0.22 µM filter and kept at -20°C until use.

### Example 2. Method for enriching populations of CSCs

The cells from both established tumor cell lines and from primary cultures of samples from cancer patients, which will be subjected to differential trypsinization, were seeded in 150 mm culture plates at a maximum of 80-90% confluence in conventional medium.

### First trypsinization:

After withdrawing the medium, the cultures were washed twice with PBS, preventing said PBS from falling directly onto the cells. 2.5 mL of diluted trypsin (T4049 sigma) at 0.05% in PBS were added. It was incubated for 2 minutes at 37°C (this time can range depending on the adhesion capacity of the line) and was subsequently inactivated with FBS, adding it slowly and preventing it from falling directly onto the cells to prevent them from becoming detached due to mechanical action.

### Seeding in conditioned medium

These obtained trypsin-sensitive cells (TS) were centrifuged and seeded in the conditioned medium (MC) described in the example 1.

### Second trypsinization

Subsequently, the cells that were stuck in the plate were washed with PBS and a second trypsinization was carried out with the diluted trypsin described above. They were incubated for 3 minutes (time varying depending on the cell line) at 37°C, trypsin was inactivated with FBS and these cells were discarded. It was washed again with PBS twice to completely remove the cells in suspension and the remainders of serum. The remaining trypsin-resistant cells (TR) became completely detached with 0.25% trypsin.

It was then checked by means of phenotypic and functional characterization that the cells obtained in the first trypsinization presented a higher percentage of CSCs than those obtained in the second trypsinization, and that the resistant cells are a more differentiated subpopulation than the general population.

Aldehyde dehydrogenase activity was studied for the phenotypic characterization (ALDEFLUOR® Kit, Stem Cell 01700), demonstrating that it is higher in the subpopulation of TS cells (94.35±1.77%) and lower in TR cells (0.4±%) compared with the general population (7.55±0.78%) (Figure 1A). With respect to the "side population", which represents the population with capacity to expel Hoescht 33342 (B2261 Sigma), and therefore expresses multidrug resistance (MDR) transporters, said population greatly increases in TS cells (70.50±4.38%) compared to TR cells (8.05±0.35) and to the general population (8.75±0.64) (Figure 1B).

As regards the capacity to form spheres after 6 days in low-adherence plates and conventional sphere medium (DMEM:F12, penicillin/streptomycin, B27, ITS, hydrocortisone, heparin, EGF, FGF), said capacity is likewise greater in TS cells (213.50±2.12) compared to TR cells (71.5±0.71) and the general population (134.00 ±38.18), furthermore being of a smaller size in the latter two (Figure 1 C).

### Example 3. Enrichment studies

Established colon tumor cell line (HCT 116) and melanoma tumor cell line (A375) were used for this study. Said lines were cultured in conventional medium, and when they reached a confluence of 80-90% sorting was performed by means of flow cytometry (cell sorting) according to the aldehyde dehydrogenase activity (ALDEFLUOR® Kit, Stem Cell 01700) as a CSC marker.

Two different populations were obtained with this technique, i.e., the cells positive for the mentioned enzyme (CSCs) and the negative cells (differentiated cells), which were seeded in low-adherence plates and sphere medium for 2-3 days. Then three types of culture conditions are established for each cell type:
Positive sorter cells (S+):
   - HCT-A375 S+: low-adherence plates and sphere medium.
   - HCT-A375 S+ T: low-adherence plates, sphere medium and a "transwell" chamber on which MSCs were seeded 48 hours prior.
   - HCT-A375 S+ MC: low-adherence plates and conditioned medium.
Negative sorter cells (S-):
   - HCT-A375 S-: low-adherence plates and sphere medium.
   - HCT-A375 S- T: low-adherence plates, sphere medium and a "transwell" chamber on which MSCs were seeded 48 hours prior.
   - HCT-A375 S- MC: low-adherence plates and conditioned medium.

The same day cell sorting was performed a general population culture flask was trypsinized, and after washing the cells twice with PBS to remove any remainder of conventional medium they were seeded in low-adherence plates and sphere medium (population referred to as NS). After this first phase, at the same time as the positive and negative populations, this population was split up to establish three different culture conditions:
- HCT-A375 NS: low-adherence plates and sphere medium.
- HCT-A375 NS T: low-adherence plates, sphere medium and a "transwell" chamber on which MSCs were seeded 48 hours prior.
- HCT-A375 NS MC: low-adherence plates and conditioned medium.

This population provides information about how the different culture conditions affect the proportion of CSCs without having to perform a prior cell sorting.

Finally, a control population was established where the cells were cultured in adherence:
- HCT-A375: culture in adherence and conventional medium.
- HCT-A375 T: culture in adherence, conventional medium and a "transwell" chamber on which MSCs were seeded 48 hours prior.
- HCT-A375 MC: culture in adherence and conditioned medium. 24 hours after seeding the cells in the wells they are washed twice with PBS and conditioned medium is added.

Every 48-72 hours the formed spheres were broken up to prevent them from becoming necrotic. To that end, the cells were collected and centrifuged, and then incubated for 5 minutes a 37°C with trypsin, which was inactivated with serum, so it was later necessary to wash with PBS to remove any remainder of serum, the cells being placed in the three low-adherence and serum-free medium culture conditions again. The medium was changed for the population growing in adherence at the same time trypsinization operations were performed. After 10-12 culture days on average, different tests were conducted to see how culture conditions affected enrichment in CSCs.

Aldehyde dehydrogenase activity was studied for the phenotypic characterization, and characteristic CSC markers of each tumor type, such as CD326 and CD44 for cell line HCT 116 and CD44 for cell line A375, were furthermore used. Two weeks later, it could be seen how in the control populations of both lines there was a significant increase in the three markers, this being more evident in cultures using the serum-free conditioned medium, such as HCT MC and HCT NS MC, where there is a two-fold increase in the percentage. The positive sorter cells (S+) maintain their characteristics after this time, and the negative sorter cells (S-) gradually acquire characteristics of CSCs, the increase being more significant with the culture with conditioned medium (S-MC) (Figure 3 A-B), so this medium favors the phenotype of the progenitor cells that are starting to differentiate not reverting to CSCs.

These results are consistent with the size of the spheres formed in the different culture conditions, being larger in the culture with conditioned medium (Figure 3 C-D).

This same experiment was performed with fresh colon tumors from different patients and in lowpass (P4) and long-pass (P8), the obtained results being equivalent to those obtained in the established tumor cell lines. It is in control populations with conditioned medium where there is a greater CSC enrichment (C MC and NS MC), the positive sorter population (S+) maintains the enrichment and the negative sorter population (S-) in the culture in sphere medium showed very little or no labeling, said labeling increasing with use of the conditioned medium (Figure 4).

The study of the cell cycle in the different culture conditions demonstrated that in the colon cancer tumor cell line HCT-116 the conditioned medium in the adherent cells increased phase G2/M at the expense of phases G0/G1 and the S with respect to the cells cultured with DEMEM and fetal serum. Nevertheless, both in the non-selected cells cultured with conditioned medium (NS-MC) and in S+ and S-, the proportion of same that are in phase G0/G1 increased compared with the respective controls (Figure 5 A-B). A somewhat different behavior was found in melanoma cells A375 which can be explained due to the enormous metastatic capacity and aggressiveness thereof.

The degree of apoptosis in the different established culture conditions was also measured and it could be seen how both complete apoptosis, such as the necrosis, gradually increases as culture conditions become more restrictive for CSCs, the values being higher in cultures in which conditioned medium was used. Since these conditions are those that most favor the culture of CSCs, culture with serum-free medium and without anchoring, the differentiated daughter cells were not in their most optimal conditions to continue with the cell cycle, entering apoptosis or necrosis (Figure 5C-D).

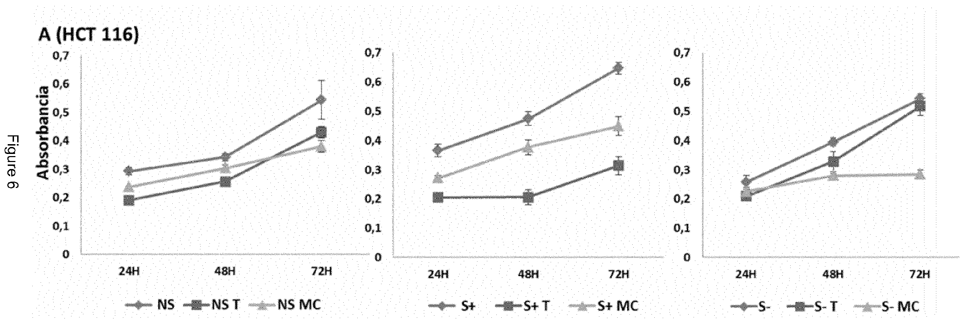

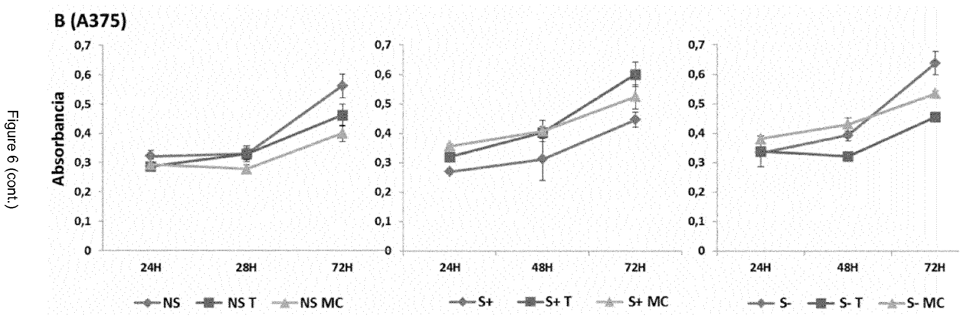

A cell proliferation experiment was conducted for cultures in suspension, and consistent with the results seen in the cell cycle, the least restrictive cultures in which conventional sphere medium and low-adherence plates (NS, S+ and S-) was used there was greater proliferation that corresponds with the increase in the division in the study of the cell cycle (Figure 6).

A genetic study was additionally conducted for cell line HCT 116, in which the genes characteristic of pluripotency were analyzed (Nanog, Sox 2, Oct 3-4 and KLF4). It can be seen in the results how expression of at least three of the genes increases in the control cultures in which conditioned medium (HCT MC and HCT NS MC) was used. There is a significant increase in the expression of the four genes in the positive sorter cell population with conditioned medium (S+ MC), and there is even a significant increase for the Oct 3-4 gene in the negative sorter cell population (S- MC), where phenotypic markers for CSCs have been emerging (Figure 7).

To complete this phase of the study an *in vivo* assay was conducted with immunosuppressed mice. First the cells were cultured for 12 days in the following conditions:
- HCT-A375 RAT: culture in adherence and conventional medium.
- HCT-A375 NS MC: low-adherence plates and conditioned medium.
- HCT-A375 S+ MC: low-adherence plates and conditioned medium.
- HCT-A375 S-: low-adherence plates and sphere medium.
- HCT-A375 S- MC: low-adherence plates and conditioned medium.

After this time, the cultures were trypsinized and a suspension of PBS was inoculated with 10,000 cells. Once the tumor developed, it was removed and broken down mechanically and enzymatically with collagenase I, collagenase IV and dispase for 30 minutes at 37°C. The cells obtained with this process were placed in culture in normal adherence culture flasks and with conventional culture medium (DMEM, 10% FBS, 1% penicillin/streptomycin). One week into the culture, cytometry was performed to check for characteristic CSC markers of each cell line studied up until now. It could be seen in the results how the markers gradually increased as the culture conditions became more restrictive for CSCs, and it is surprising how the negative sorter cells, which were in conditioned medium before being inoculated in the mouse, presented one of the highest enrichments. This result is consistent with the results of the phenotypic labeling experiments after 12 days in culture (Figure 3 and 4) and with the expression of pluripotency genes (Figure 7), indicating that the conditioned medium favors regression to CSCs of the progenitor cells that were in the first phases of differentiation, and it favors them proliferating to restore a tumor in the mouse.

### Example 4.- ANALYSIS OF THE CSCs ENRICHMENT MEDIUM:

Based on prior the studies aimed at analyzing which culture condition is the most suitable for greater CSC enrichment, it can be concluded that the use of the conditioned medium is what offers the greatest advantages, so the characteristics of this medium will be further analyzed below:
The first study focused on concentrating the conditioned medium to see if better results in CSC enrichment are obtained. To do so, special Millipore filters concentrating the medium 25-fold by means of centrifugation were used: Amicon Ultra-15 centrifugal filter units (3 kDa). Once the conditioned medium was obtained and passed through a 0.22 µM filter, 10 ml of this medium were taken and centrifuged in the Amicon tubes, leaving a final volume of 1 ml. Then different culture concentrations were established for cell line HCT 116 by diluting the concentrated conditioned medium (25%, 50%, 75% and 100%), and they were kept in culture for 6 days in low-adherence plates, performing trypsinization every 28-72 hours to break up the formed spheres. Once this time went by, the cells were subjected to phenotypic labeling for aldehyde dehydrogenase, CD 44 and CD 326. The greatest enrichment was obtained in the cell culture with the medium concentrated to 100%, obtaining a 4-fold increase for aldehyde dehydrogenase, 22-fold increase for CD 44 and 8-fold increase for CD 326, with respect to the control line growing in adherence and conventional medium (HCT), and a 2-fold increase for enzyme, 17-fold increase for CD 44 and 8-fold increase for CD 326, which is greater than the line in culture in low-adherence plates and sphere medium (HCT ME) (Figure 9).

Then Luminex technology was applied to analyze the composition of the conditioned medium and to see which elements had the most weight in enriching CSCs. To that end, the culture conditions from the preceding paragraph were established for the established tumor cell line HCT 116 and for two fresh colon tumor cell lines (C3 and C4). The supernatant was collected after 12 days in culture and passed through a 0.22 µM filter, and then the Luminex test was conducted, analyzing 18 different cytokines. It was possible to extract, from the results of this test, four interleukins (IL6, IL8, IL12 and IL23) and seven factors (EGF, FGF, HGF, GMCSF, MCSF, PIGF1 and VEGF), which vary in different culture conditions, and the value of which is higher in the culture with conditioned medium (see Table 1).

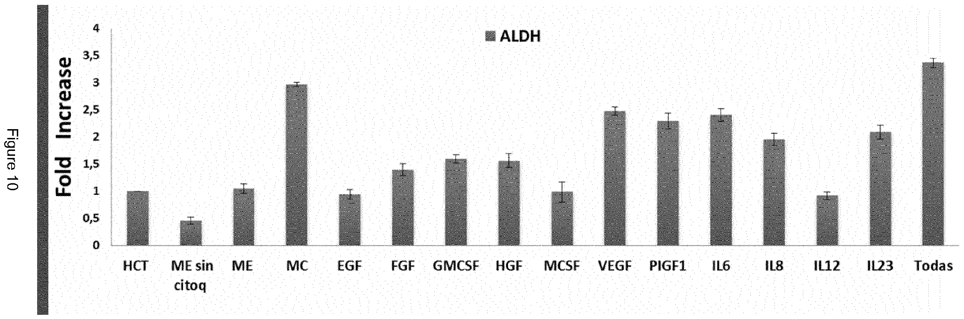

Then a test was conducted to analyze what effect each cytokine has individually. It was placed in culture in low-adherence plates and in the sphere medium, but modifying the cytokines that are added. To that end, a medium was used as a starting medium with the following components (sphere medium without EGF or FGF): DMEM-F12; 1% streptomycin-penicillin; 1 µg/mL hydrocortisone; 4 ng/mL heparin; 10 µg/mL insulin; 1X B27 and furthermore a specific cytokine. The medium was kept in culture for 6 days and after this time period it was possible to see the existence of cytokines producing greater enrichment in CSCs, measured by aldehyde dehydrogenase activity, than those cytokines normally used for the sphere medium (EGF and FGF) (Figure 10).

Based on these results of the use of each cytokine individually, different combinations were established to see how they interact. To that end, 16 different combinations were established, and these were added to the sphere medium without EGF or FGF, mentioned above (DMEM:F12, penicillin/streptomycin, B27, ITS, hydrocortisone, heparin). After 6 days in culture a test to determine aldehyde dehydrogenase activity was conducted and it could be seen that the worst two combinations (PIGF1-IL23 and VEGF-IL23) cause the exact same increase in the percentage of CSCs with respect to the control population (HCT) as the usual combination of EGF and FGF (HCT ME). The remainder of combinations generated a much higher enrichment, but it is important to mention factor HGF, which when combined with IL6 causes a 2.5-fold increase, and combined with IL12 and IL23 causes a 2-fold increase with respect to the control population (HCT) and the sphere medium (HCT ME) (Figure 11). Therefore, any combination based on the cytokines set forth in Figure 11 entails a higher enrichment in CSCs.

The concentration of the conditioned medium at 75% and at 100% (Figure 9) thereby allows better enrichment when using the MC processed from the MSCs.

## Claims

1. A cell culture medium suitable for isolating and/or enriching cancer stem cells (CSCs), obtained or obtainable by a method comprising the following steps:
a. Seeding mesenchymal stem cells (MSCs) in a plate, vessel or flask suitable for culturing cells using a culture medium suitable for this purpose;
b. Optionally removing any remainder of medium and/or serum from the culture medium from step a) by means of washing;
c. Adding a sphere medium or conventional medium without FBS to the product of step b);
d. Collecting the culture medium from step c) and adding new sphere medium;
e. Repeating the process from step d) until the cells in culture reach a level of confluence of 80-90%;
f. Optionally filtering and freezing the medium obtained in steps d) and e) until use.

2. The cell culture medium according to claim 1, wherein said medium is obtained or obtainable by a method comprising the following steps:
a. Seeding mesenchymal stem cells in a plate, vessel or flask suitable for culturing cells using a conventional culture medium, where said cells have a confluence between 40% and 90% at the start of the process;
b. Removing any remainder of medium and/or serum from the culture medium from step a) by means of washing at least 24 hours after the start of the process;
c. Adding a sphere medium or conventional medium without FBS to the product of step b);
d. Collecting the culture medium from step c) at least 48 hours after adding the medium in step c), and adding new sphere medium;
e. Repeating the process from step d) until the cells in culture reach a level of confluence of 80-90%;
f. Optionally filtering, preferably using a 0.22 µM filter, and freezing the medium obtained in steps d) and e) until use.

3. A method for obtaining a cell culture medium suitable for isolating and/or enriching cancer stem cells (CSCs), comprising the following steps:
a. Seeding mesenchymal stem cells (MSCs) in a plate, vessel or flask suitable for culturing cells using a culture medium suitable for this purpose;
b. Optionally removing any remainder of medium and/or serum from the culture medium from step a) by means of washing;
c. Adding a sphere medium or conventional medium without FBS to the product of step b);
d. Collecting the culture medium from step c) and adding new sphere medium;
e. Repeating the process from step d) until the cells in culture reach a level of confluence of 80-90%;
f. Optionally filtering and freezing the medium obtained in steps d) and e) until use.

4. A method for obtaining a cell culture medium suitable for isolating and/or enriching cancer stem cells (CSCs), comprising the following steps:
a. Seeding mesenchymal stem cells in a plate, vessel or flask suitable for culturing cells using a conventional culture medium, where said cells have a confluence between 40% and 90% at the start of the process;
b. Removing any remainder of medium and/or serum from the culture medium from step a) by means of washing at least 24 hours after the start of the process;
c. Adding a sphere medium or conventional medium without FBS to the product of step b);
d. Collecting the culture medium from step c) at least 48 hours after adding the medium in step c), and adding new sphere medium;
e. Repeating the process from step d) until the cells in culture reach a level of confluence of 80-90%;
f. Optionally filtering, preferably using a 0.22 µM filter, and freezing the medium obtained in steps d) and e) until use.

5. The medium obtained by the method of either of claims 3 or 4 and concentrated using centrifugation and/or filters.

6. A natural or synthetic culture medium suitable for isolating and/or enriching cancer stem cells (CSCs) obtainable or obtained according to the method of either of claims 3 or 4, additionally **characterized in that** it comprises:
a. a medium suitable for cell culture, such as a conventional medium or a sphere medium; and
b. at least one compound selected from the list consisting of: IFNβ, IFNγ, IL10, IL2, IL4, IL5, IL6, IL8, IL12, IL23, EGF, FGF, HGF, GMCSF, MCSF, PEGFBB, TNFα, PIGF1 and VEGF, supplementing the medium from paragraph a).

7. The natural or synthetic culture medium according to claim 6, wherein the compound of paragraph b) supplementing the medium is selected from the list consisting of any of the following combinations of compounds:
a. IL6, IL8, IL12 and IL23;
b. IL6, IL8, IL12, IL23, EGF and FGF;
c. GMCSF, MCSF, HGF, PIGFI and VEGF;
d. EGF, FGF, GMCSF, MCSF, HGF, PIGFI and VEGF;
e. IL6 and GMCSF;
f. GMCSF and IL12;
g. GMCSF and IL23;
h. IL6 and HGF;
i. HGF and IL12;
j. HGF and IL23;
k. PIGFI and IL6;
l. PIGFI and IL12;
m. PIGFI and IL23;
n. VEGF and IL6;
o. VEGF and IL12;
p. VEGF and IL23;
q. IL6, IL8, IL12, IL23, EGF, FGF, GMCSF, MCSF, HGF, PIGFI and VEGF.

8. The natural or synthetic culture medium according to claim 6, wherein the compounds of paragraph b) supplementing the medium comprise the combination of compounds shown in Table I.

9. The natural or synthetic culture medium according to any of claims 6 to 8, wherein said medium suitable for cell culture is selected from the list consisting of: Eagle's Basal Medium (EBM), minimum essential medium (MEM), Dulbecco's Modified Eagle's Medium (DMEM), medium 199, Ham F-10, Ham F-12, McCoy's 5A, Dulbecco's MEM/FI2, RPMI 1640 medium, and Iscove's Modified Dulbecco's Medium (IMDM), and comprising 10% FBS (fetal bovine serum and 1% penicillin/streptomycin).

10. The natural or synthetic culture medium according to any of claims 6 to 8, wherein said medium suitable for cell culture comprises DMEM-F12 (Dulbecco's Modified Eagle's Medium/Nutrient Mixture F-12 Ham Sigma-D8437).

11. The natural or synthetic culture medium according to any of claims 6 to 8, wherein said medium suitable for cell culture comprises DMEM-F12; streptomycin-penicillin; hydrocortisone; heparin; insulin; and 1X B27.

12. The natural or synthetic culture medium according to any of claims 6 to 8, wherein said medium suitable for cell culture comprises DMEM-F12 and wherein the compounds of paragraph b) supplementing the medium comprise the combination of compounds shown in Table I.

13. A concentrated conditioned medium obtained by centrifuging and/or filtering any of the media as they are defined in any of claims 6 to 12.

14. A method for enriching cancer stem cells (CSCs) in a cell culture comprising the following steps:
a. Seeding a biological sample comprising CSCs in the medium defined in any of claims 1 to 2 or 5 to 13; and
b. Obtaining the population of cells enriched in CSCs.

15. A method for enriching CSCs in a cell culture wherein, before or after the method described in claim 14, the following steps are performed:
a. Obtaining a biological sample comprising a cell culture of cancer stem cells (CSCs), whether they are obtained from the population from step b) of claim 14 or not, adhered to a plate, vessel or flask suitable for culturing cells;
b. Withdrawing the culture medium from the biological sample from step a) and optionally washing the cell culture;
c. Adding diluted trypsin, preferably in PBS, at a concentration between 0.01 and 1%, to the primary culture from step b) and incubating said cells with the diluted trypsin;
d. Inactivating the trypsin; and
e. Obtaining those cells that have become detached from the medium due to the action of trypsin.

16. A cellular composition obtained by the method for enriching according to any of claims 14 to 15.

17. A population of cells obtained by the method for enriching according to any of claims 14 to 15.

18. A substantially pure population of cells obtained by the method for enriching according to any of claims 14 to 15.

19. Use of the natural or synthetic culture medium according to any of claims 1 to 2 or 5 to 13 for isolating and/or enriching cancer stem cells.
